# EUROPEAN PATENT SPECIFICATION

(11) **EP 1 265 649 B1**
(45) Date of publication and mention of the grant of the patent: **23.02.2005**
(21) Application number: 01920439.5
(22) Date of filing: 16.03.2001
(51) Int. Cl.: A61L 15/60

(54) **PERMANENTLY WETTABLE SUPERABSORBENT FIBERS**
PERMANENT WASSERBENETZBARE HOCHABSORBIERENDE FASERN
MATERIAUX SUPERABSORBANTS A MOUILLABILITE PERMANENTE

(30) Priority: 21.03.2000 US 531247
(43) Date of publication of application: 18.12.2002
(73) Proprietor: KIMBERLY-CLARK WORLDWIDE, INC., Neenah, Wisconsin 54956 (US)
(72) Inventor: QIN, Jian, Appleton, WI 54915 (US); ZHANG, Xiaomin, Appleton, WI 54911 (US); RANGANATHAN, Sridhar, Suwanee, GA 30024 (US); LI, Yong, Appleton, WI 54915 (US)
(74) Representative: Beacham, Annabel Rose
(86) International application number: PCT/US2001/008472
(87) International publication number: WO 2001/070287

(56) References cited:
- EP-A- 1 125 494
- US-A- 3 989 586
- US-A- 5 151 465

## Description

### Technical Field

The invention relates to superabsorbents, more particularly to superabsorbent fibers that are permanently wettable.

### Background of the Invention

The use of water-swellable, generally water-insoluble absorbent materials, commonly known as superabsorbents, in disposable absorbent personal care products is well known. Such absorbent materials are generally employed in absorbent products such as diapers, training pants, adult incontinence products, and feminine care products in order to increase the absorbent capacity of such products while reducing their overall bulk. Absorbent materials are generally present as superabsorbent particles in a fibrous matrix, such as a matrix of wood pulp fluff.

Superabsorbent particles are sometimes difficult to use because they do not remain stationary during the manufacturing process and may shift position in the article. The potential advantages of using superabsorbent fibers, as opposed to superabsorbent particles, include improved product integrity, better containment, and improved absorbent properties, such as rapid fluid absorption and fluid distribution properties. The use of superabsorbent fibers may also lead to improved product attributes, such as thinner and softer products that provide a better fit, less gel migration, and potential simplification of product manufacturing processes.

The surface characteristics of superabsorbent fibers play a central role in determining the fluid handling properties of composites that contain them. Accordingly, there is a need for superabsorbent fibers having sufficient surface wettability. Commercially available superabsorbent fibers do not exhibit permanent wettability.

US-A-3 989 586 discloses a method of making paper products having increased absorbency. A surfactant is employed in order to disperse a copolymer in a cellulosic web or pulp and is added to the copolymer before the copolymer is converted into a superabsorbent material. The surfactant solution is then removed from the pulp by drying before the superabsorbent material is formed by treatment with alkali.

### Summary of the Invention

The invention includes methods of making permanently wettable superabsorbent material involving treating the superabsorbent material with a surfactant solution. A surfactant is used that has at least one functional group that is reactive with the superabsorbent material and at least one non-reactive and hydrophilic functional group. The surfactant is applied to the superabsorbent material when functional groups on the surface of the superabsorbent material are in an activated state. In one embodiment, the surfactant is applied to the superabsorbent material when the superabsorbent material is solvated. Desirably, the surfactant solution includes a solvent that is a solvent to the surfactant but a non-solvent to the superabsorbent material and the surfactant solution includes an amount of water sufficient to solvate the surface of the superabsorbent material but less than sufficient to cause significant swelling of the superabsorbent material.

The invention further includes permanently wettable superabsorbent materials, such as fibers, made by the method and disposable absorbent products comprising the permanently wettable superabsorbent material. The permanently wettable superabsorbent materials made by the method have a floating time less than 30 seconds and cause a reduction in surface tension of saline less than about 30%.

An object of the invention is to provide superabsorbent fibers, and methods of making same, exhibiting a permanently wettable surface (θ < 90° and floating time of less than 30 seconds) and causing a low reduction in surface tension of fluid, desirably less than or equal to about 30% reduction in surface tension of saline (0.9% NaCl), more desirably less than or equal to about 25%, and more desirably 20%, reduction in surface tension of saline.

### Detailed Description of the Invention

Superabsorbent fibers (SAFs) have the potential for better fluid distribution properties than superabsorbent particles (SAPs) due to the smaller dimension and larger surface area of fibers. However, commercially available SAFs, such as Fiberdri® from Camelot Superabsorbents Ltd. of Calgary, Canada and Oasis® from Technical Absorbents UK, are actually worse in fluid distribution properties than commercially available SAPs. One commonly advanced reason for this poorer performance is the faster fluid pickup rate of superabsorbent fibers. However, there are at least two other causes of this inferiority. First, the surface of a SAF is made hydrophobic during the fiber spinning process. Second, surfactants applied to the fiber to counteract the fiber's hydrophobicity may be released from the fiber into liquid that comes in contact with the fiber, and lower the surface tension of the liquid.

Disclosed herein are permanently wettable (hydrophilic) superabsorbent fibers which do not cause a significant reduction in saline surface tension when placed in saline, and methods to modify superabsorbent fibers into such materials. The term "permanent" as used herein does not necessarily mean that the superabsorbent remains wettable for an indefinite period of time but rather means that the superabsorbent remains wettable at least upon repeated washings and upon normal usage. Composites containing the permanently wettable superabsorbent fibers exhibit improved fluid distribution properties over composites containing other SAFs. While the invention is described herein particularly as a method to make permanently wettable superabsorbent fibers, it should be understood that it is also applicable to making other forms of permanently wettable superabsorbent materials such as, for example, a particulate, a film, a nonwoven, a bead, a foam, and a coform.

The belief that superabsorbent fibers should exhibit improved fluid distribution over SAPs is based on the Laplace equation: p = 2 γ cos θ / R_{c} (where p is capillary pressure, γ is surface tension of fluid, θ is the contact angle at the liquid-solid-air interface, and R_{c} is the capillary radius). Due to a significant reduction in the capillary radius, R_{c}, from SAPs to SAFs, the capillary pressure p should have a significant increase, which means an increase in fluid distribution power. However, SAFs have actually been found to perform worse than SAPs in fluid distribution in some cases.

Without meaning to be held to theory, it is believed that the reason for the poorer results is that the SAF surface is hydrophobilized during the spinning process. For example, sodium polyacrylate, a commonly used superabsorbent material, is a hydrophilic polymer due to the presence of carboxyl groups (-COO⁻) and carboxylic acid groups (-COOH). However, the surface of a sodium polyacrylate fiber can be very hydrophobic if a solution of the polymer is dried in hot air. That is because hot air is hydrophobic relative to water so that hot air attracts more hydrophobic segments (-C-C-)ₙ of the sodium polyacrylate onto the surface and at the same time repels the hydrophilic segments (-COO⁻, -COOH) of the polymer away from the surface. This is so-called surface hydrophobilization. Surface hydrophobicity is one of the reasons why SAFs do not exhibit improved fluid distribution properties. A hydrophobic surface is one with a contact angle θ greater than 90°, which results in a negative capillary pressure in the Laplace equation.

In order to make wettable superabsorbent fibers, others have applied surfactant onto the fibers to improve their contact angle and wettability. This approach can solve the surface wettability problem but creates another problem. The surfactant applied does not permanently stay on the surface of the superabsorbent fibers due to the surfactant used and the methods of application. The surfactant on the surface of superabsorbent fibers is fugitive and will be dissolved into the fluid contacting the fiber, which dramatically reduces the surface tension γ of the fluid and negatively hinders fluid distribution properties.

The methods of the invention involve application of a reactive surfactant to the surface of a superabsorbent fiber when the fiber is activated. There are several ways to achieve this. In a desired method particularly described herein, the surfactant is applied to the fiber when the fiber is solvated. The surfactant is applied to the fiber in a liquid that is a solvent for the surfactant but not for the fiber. Water is added to the surfactant solution. The amount of water should be enough to solvate the surface of the fiber so that ionic groups on the surface macromolecules of the fiber can be freed to rotate to promote interaction with the functional reactive groups of the surfactant. However, the amount of water should not be enough to cause significant swelling of the fiber. The fiber can be treated with the surfactant/water/solvent solution in a number of ways, including spraying or immersion. The treated fiber can be washed, if desired, and then dried to remove the solvent and water.

Other methods can be used to promote interaction between the functional groups of the surfactant and the SAF. For example, the SAF can be exposed to a higher humidity environment for a while and then treated with the surfactant. The water vapor will solvate the surface of the SAF to achieve the same solvated state as the liquid water. Another example is that the SAF can be exposed to a high energy radiation, such as e-beam or plasma, and then treated with the surfactant. Such radiation can activate the surface macromolecules, generating free radicals or ions, which promotes reaction with the surfactant.

### Fibers

Any of a number of superabsorbent fibers can be used in the invention. As used herein, the term "superabsorbent" refers to a water-swellable, water-insoluble material capable, under the most favorable conditions, of absorbing at least about 10, desirably of about 20, and often of up to about 1000 times its weight in water. Organic materials suitable for use as a superabsorbent material of the present invention may include natural materials such as agar, algin, carrageenan, starch, pectin, guar gum, chitosan, and the like, modified natural materials such as carboxyalkyl cellulose, methyl cellulose, hydroxyalkyl cellulose, chitosan salt, dextran, and the like; as well as synthetic materials, such as synthetic hydrogel polymers. Such hydrogel polymers include, but are not limited to, alkali metal salts of polyacrylic acids, polyacrylamides, polyvinyl alcohol, ethylene maleic anhydride copolymers, polyvinyl ethers, hydroxypropylcellulose, polyvinylmorpholinone, and polymers and copolymers of vinyl sulfonic acid, polyacrylates, polyacrylamides, polyvinyl amines, polyallylamines, polyvinylpyrridine, and the like. Other suitable polymers include hydrolyzed acrylonitrile grafted starch, acrylic acid grafted starch, and isobutylene maleic anhydride copolymers and mixtures thereof. The hydrogel polymers are desirably lightly crosslinked to render the material substantially water insoluble. Crosslinking may, for example, be by irradiation or by covalent, ionic, van der Waals, or hydrogen bonding.

In one embodiment of the present invention, the absorbent fibers comprise one or more superabsorbent materials in the form of a sodium salt of a cross-linked polymer. Such superabsorbent materials include, but are not limited to, Fiberdri® 1161, Fiberdri® 1231, and Fiberdri® 1241 (all available from Camelot Superabsorbent Ltd. of Calgary, Canada); and Oasis@ 101, Oasis® 102, and Oasis@ 111 (all available from Technical Absorbents, UK).

The superabsorbent fibers can be made by a number of methods known to those skilled in the art.

### Surfactants

Suitable surfactants are compounds having at least one functional group reactive with the SAF and at least one non-reactive and hydrophilic functional group. Reactive functional groups include cationic groups for an anionic SAF and anionic groups for a cationic SAF. Examples of cationic groups are, without limitation, quaternary ammonium groups, and amino groups when the anionic SAF contains acidic groups such as carboxylic acid groups. Examples of anionic groups are, without limitation, carboxyl groups, sulfonate groups, phosphate groups, and their corresponding acid groups when the cationic SAF contains basic groups such as amino groups.

Non-reactive, hydrophilic functional groups include, without limitation, hydroxyl groups, ether groups, carboxylic acid groups, amino groups, and imino groups.

One suitable surfactant is Rhodamox LO (lauryl dimethylamine oxide) from Rhone-Poulenc, Inc. The surfactant solution desirably has a concentration of about 0.001 g to 20 g surfactant per 1000 g solvent, more desirably about 0.005 g to 10 g surfactant per 1000 g solvent, more desirably about 0.01 g to 5 g surfactant per 1000 g solvent, and more desirably about 0.05 to 1 g surfactant per 1000 g solvent.

### Solvents

The solvent has to be compatible or miscible with the SAFs' activating agent, such as water. The solvent is one which solvates the surfactant but does not substantially solvate the fiber. Appropriate solvents can be selected by those skilled in the art and include, without limitation, isopropanol, methanol, ethanol, butyl alcohol, butanediol, butanetriol, butanone, acetone, ethylene glycol, propylene glycol, glycerol, and mixture of the above. Preferred solvents include isopropanol, ethanol, and acetone.

### Amount of Water

The amount of water to be added is important; it should be enough to solvate the surface of the fiber so that ionic groups on the surface macromolecules of the fiber can be freed to rotate to promote interaction with the functional groups of the surfactant. However, the amount of water should not be enough to cause significant swelling of the fiber. Significant swelling of a SAF is defined as a volume increase of at least about 100%.

A desired amount of water is from 0.5 to 30 weight % by total weight of the solvent, desirably from about 1 to 20%, more desirably from about 1 to 15%, most desirably from about 1 to 10%. All percentages herein are by weight unless otherwise stated.

While not meaning to be limited to theory, the water is believed to act as an activation agent to promote reaction between the reactive functional groups of the surfactant and the functional groups of the superabsorbent fiber. For example, if a cationic/non ionic surfactant is applied onto the surface of polyacrylate superabsorbent fiber in the absence of water, anionic groups (-COO⁻) of the fiber are not present on the surface due to the surface hydrophobilization or are not in the ionic form due to lack of water. Though the surfactant has cationic groups they cannot form ionic bonds with the anionic groups of the fiber. The surfactant just adheres to the surface of the fiber and becomes fugitive. However, when a certain amount of water is present in the treating solution, the water can solvate the surface of the fiber and the anionic groups near the surface of the fiber will be able to rotate from an inward conformation to an outward conformation which allows the cationic groups of the surfactant to form ionic bonds with these outward anionic groups and achieve a permanent surface wettable treatment.

### Reaction Conditions

The fiber can be treated with the surfactant solution in a number of ways, including spraying or immersion. The ratio of SAF to treating solution will vary greatly depending upon how the surfactant is applied to the fiber. For example, a ratio of 1:1 (grams of SAF to grams of solution) to 1:5 is desirable when the solution is applied by spraying. In one embodiment of immersion treatment, the SAF is added into the pre-prepared treating solution. The ratio of SAF to treating solution will desirably range from about 1:1 to 1:500, more desirably from about 1:1 to 1:100, more desirably from about 1:1 to 1:50.

The treatment is carried out at a temperature from about 0°C to 100°C, more desirably from about 10 to 60°C, more desirably about 20 to 30°C, desirably about room temperature (23°C). The length of treatment depends upon the method of application, the temperature of application, as well as the components. The length of treatment will range from about 0.01 to 1 hour, desirably from about 0.05 to 0.5 hour, more desirably from about 0.1 to 0.3 hour, desirably with constant agitation.

### Washing and Drying

The purpose of washing is to remove any fugitive surfactant. Even when a reactive surfactant and proper reaction conditions are selected, fugitive surfactant can still be generated when too much surfactant is used (number of surfactant molecules is more than number of available functional groups on the surface of the SAF or there is incomplete reaction between the surfactant and the functional groups of the SAF). An effective washing applies a weight ratio of SAF to washing solvent in a range of about 1:2 to 1:500, desirably about 1:5 to 1:200, more desirably about 1:10 to 1:100. Washing at room temperature is preferred, however, a temperature ranging from 0°C to 100°C can be used. A mixing aid, such as mechanical agitation, vibration, or ultrasonic treatment, will help to achieve a higher effectiveness of the washing. The liquid used for washing will be a solvent for the surfactant but not for the fiber.

Any conventional drying method, such as air drying at ambient condition or at an elevated temperature, vacuum drying, freeze drying, supercritical drying, etc., can be used to dry the treated and/or washed fibers.

The superabsorbent fibers of the present invention are suitable for use in disposable absorbent products such as personal care products, such as diapers, training pants, baby wipes, feminine care products, adult incontinent products, and medical products, such as wound dressings, surgical capes, and drapes.

The SAFs can be used in woven and nonwoven products as commercially available superabsorbent fibers are now used. It may be desirable to mix the inventive superabsorbent fibers with other fibers and/or to add superabsorbent particles to a web made of the inventive fibers to make an absorbent structure. The invention disclosed can also be applied to other forms of superabsorbents, such as particulates, films, flakes, nonwovens, beads, and foams to improve their surface wettability.
In one embodiment of the present invention, a disposable absorbent product is provided, which includes a liquid-permeable topsheet, a backsheet attached to the topsheet, and an absorbent structure made with the inventive fibers positioned between the topsheet and the backsheet.

Disposable absorbent products, according to all aspects of the present invention, are generally subjected during use to multiple insults of a body liquid. Accordingly, the disposable absorbent products are desirably capable of absorbing multiple insults of body liquids in quantities to which the absorbent products and structures will be exposed during use. The insults are generally separated from one another by a period of time.

Those skilled in the art will recognize materials suitable for use as the topsheet and backsheet. Exemplary of materials suitable for use as the topsheet are liquid-permeable materials, such as spunbonded polypropylene or polyethylene having a basis weight of from about 15 to about 25 grams per square meter. Exemplary of materials suitable for use as the backsheet are liquid-impervious materials, such as polyolefin films, as well as vapor-pervious materials, such as microporous polyolefin films.

The invention is further illustrated by the following examples, which are not to be construed in any way as imposing limitations upon the scope thereof. On the contrary, it is to be clearly understood that resort may be had to various other embodiments, modifications, and equivalents thereof, which, after reading the description herein, may suggest themselves to those skilled in the art without departing from the spirit of the present invention.

### Examples

Two properties of SAFs were measured in Example 1: floating time, to indicate surface wettability, and surface tension using a saline solution, to indicate fugitiveness of the surfactant. In Example 2, an inclined wicking test was used to evaluate fluid distribution properties of composites including the inventive SAF samples. The Free Swell, dry weight, density, and saline pick-up are also reported. The testing procedures are summarized below:
Floating Time: about 80 g of 0.9% NaCl isotonic saline, available from RICCA Chemical Co. (Arlington, TX), was placed in a 100 ml beaker. 0.01 g of superabsorbent fiber was weighed and gently added to the beaker from a height about 5 cm above the surface of the saline. The time from when the fiber first contacted the surface of the saline to the time the fiber was below the surface of the saline was recorded as Floating Time.
Surface Tension Test: One gram of superabsorbent fiber was thoroughly mixed with 150 grams of 0.9% NaCl saline in a flask. After 15 minutes, the saline was poured into a glass container. A Kruss Processor Tensiometer K 12 was used to measure the surface tension of the treated saline.
Absorbency Tests: About 0.16 g of superabsorbent fiber was weighed and placed into a plastic AUL test cylinder with a 100 mesh screen on its bottom. A plastic piston was placed on the top of the discs which generated a pressure of about 0.01 psi. The cylinder was then placed into a dish which contained about 50 ml of 0.9% NaCl saline. After 1 hour, the cylinder was taken out and placed on paper towel to blot interstitial fluid. The blotting was continued by moving the cylinder to dry paper towel until there was no fluid mark visible on the paper towel. The weight difference of the cylinder between wet and dry represented total amount of fluid absorbed by the SAF and was reported as the Free Swell Absorbency. The Absorbency Test is described in more detail in WO 99/17695 in the section titled "Flooded Absorbency Under Zero Load".
Inclined Wicking Test: Superabsorbent fiber samples were air laid with wood pulp fluff into composites having a total basis weight of 400 gsm. The composites were densified to about 0.2 g/cc and cut into a size of 33 cm by 5.1 cm. A cut sample was placed into an inclined wicking trough (inclined angle of 30°) for the wicking test. The testing fluid was 0.9% NaCl saline. The test lasted about 90 minutes and both wicking distance and wicking capacity (pick-up) were recorded as parameters to reflect fluid distribution capabilities. The Wicking Test is described in more detail in European Publication 761 192 A2 in the section titled "Wicking Parameter".

Density was determined by dividing the basis weight by the thickness of the web. The thickness was determined using a Digimatic Indicator Model 1DF-150E, available from Mitutoyo Corporation, Japan, with an applied pressure of 0.05 psi and an accuracy of 0.001 mm.

### Example 1: Formation of Wettable and High Surface Tension Fibers

A commercially available superabsorbent fiber (Fiberdri 1241-available from Camelot Superabsorbent Ltd. of Calgary, Canada) was used. This fiber is a crosslinked copolymer of maleic anhydride and isobutylene. This fiber is wettable (floating time less than 30 seconds) but causes a surface tension reduction of saline (0.9% NaCl) from 72 to 47 dyne/cm (34.7% reduction). The fiber was washed up to six times in isopropanol (weight ratio of fiber to isopropanol about 1 to 10) to remove any surfactant that came with the superabsorbent fiber. Each washing time was about 1 hour. Fresh isopropanol was added to the fiber for each washing. The results in Table 1 for samples 1-7 show that floating time of the fiber and surface tension of the saline increased with each washing, indicating that surfactant was indeed washed off of the fiber and that the surface of the fiber became more hydrophobic.

In a second step, the washed SAF was treated with a cationic-nonionic surfactant, Rhodamox LO (lauryl dimethylamine oxide), available from RhonePoulenc, Inc., in an isopropanol medium with (Sample 9) or without (Sample 8) water. The weight ratio of SAF/water/isopropanol/Rhodamox LO was 1:1:50:0.005. The treated fiber was then washed with fresh isopropanol to ensure that no unreacted surfactant was left on the surface of the fiber. The results of the floating test and surface tension tests show that the fiber treated in the presence of water was permanently wettable, with a floating time of 26.1 seconds, and maintained a surface tension of 57.5 dyne/cm (20% reduction).

**Table 1**

| **Sample No.** | **Superabsorbent Fibers** | **Floating Test (seconds)** | **Surface Tension (dyne/cm)** |
|---|---|---|---|
| 1 | Fiberdri 1241 as received | 19.1 | 47.8 |
| 2 | Fiberdri 1241 washed once | 59.5 | 54.2 |
| 3 | Fiberdri 1241 washed twice | greater than 60 | -- |
| 4 | Fiberdri 1241 washed 3x | greater than 60 | -- |
| 5 | Fiberdri 1241 washed 4x | greater than 60 | 56.9 |
| 6 | Fiberdri 1241 washed 5x | greater than 60 | 57.1 |
| 7 | Fiberdri washed 6x | greater than 60 | 58.1 |
| 8 | Fiberdri 1241 washed 6x and treated with cationic surfactant (without water) | 24.6 | 44.3 |
| 9 | Fiberdri 1241 washed 6x and treated with cationic surfactant (with water) | 26.1 | 57.5 |

### Example 2: Formation and Testing of Composites

Sample Nos. 1, 7, and 9 from Example 1 were used as fiber samples in Example 2. Sample No. 1 was selected to represent a wettable but low surface tension fiber, sample No. 7 was selected to represent a non-wettable but high surface tension fiber, and sample No. 9 was selected to represent a wettable and high surface tension sample. Each fiber sample was made into a composite through an air forming process.

The composites were made using an air former. The air former consists of two compartments. The first compartment is a cylindrical chamber having a diameter of about 2 feet and a height of about 1 foot and equipped with 10 air nozzles connecting with a source of compressed air. This chamber is for the purpose of mixing components intended to add into the composite. The compressed air will generate a strong turbulence to aid mixing. The second chamber, called the air laying chamber, has a dimension of about 1.5' (L) x 0.5' (W) x 2.5' (H) and is connected with a vacuum source. The SAF and wood pulp fluff were added into the first chamber at a certain ratio, mixed by the air turbulence and then sucked into the second chamber under vacuum through a metal screen which connects the two chambers. Fully homogenized components were air laid on a sheet of forming tissue to form the composite. The composite prepared was compressed by a Carver press at a temperature of 150°C and a pressure of 500 psi for 10 seconds.

Each composite included 60% superabsorbent fiber and 40% wood pulp fluff fiber (Coosa CR1654 produced by US Alliance Coosa Pines Corporation, Alabama), and had a basis weight of 400 grams per square meter (gsm). The composites were densified to around 0.2 g/cc using a Carver press. The composites were cut into 2" (5.08 cm) by 13" (33.02 cm) stripes and a stripe was placed into a trough having an inclined angle of 30° to the horizontal to measure inclined wicking distance and wicking capacity. Table 2 below summarizes the results.

**Table 2**

| Composite No. | SAF No. | Free Swell of SAF (g/g) | Dry Weight (g) | Density (g/cc) | Saline Pick-Up (g) | Wicking Distance (cm) |
|---|---|---|---|---|---|---|
| 1 | 1 | 30.0 | 6.66 | 0.184 | 64.6 | 15.2 |
| 2 | 7 | 30.7 | 6.40 | 0.177 | 68.5 | 15.2 |
| 3 | 9 | 29.4 | 6.75 | 0.188 | 86.6 | 18.2 |

Note the greater wicking distance and higher wicking capacity for the inventive SAF containing composite.

## Claims

1. A method of making a permanently wettable superabsorbent material, comprising:
treating the superabsorbent material with a surfactant solution;
wherein the surfactant has at least one first functional group reactive with a second functional group of the superabsorbent material and at least one non-reactive and hydrophilic functional group; and
wherein the surfactant is applied to the superabsorbent material when the second functional groups on the surface of the superabsorbent material are activated.

2. The method of claim 1, wherein the surfactant solution includes a solvent that is a solvent to the surfactant but a non-solvent to the superabsorbent material; and
wherein the surfactant solution includes an amount of water sufficient to solvate the surface of the superabsorbent material but less than sufficient to cause significant swelling of the superabsorbent material.

3. The method of claim 2 further comprising drying the treated superabsorbent material to remove the solvent and the water.

4. The method of claim 1 further comprising washing the treated superabsorbent material with a solvent to remove fugitive surfactant.

5. The method of claim 2, wherein the treatment is by immersion or spray.

6. The method of claim 1, wherein the superabsorbent material is a superabsorbent fiber.

7. The method of claim 1, wherein the superabsorbent material is in a form selected from the group consisting of a particulate, a film, a nonwoven, a bead, a foam, and a coform.

8. A permanently wettable superabsorbent fiber obtainable by the method of claim 6.

9. The method of claim 1, wherein the superabsorbent material is selected from the group consisting of alkali metal salts of polyacrylic acids, polyacrylamides, polyvinyl alcohol, ethylene maleic anhydride copolymers, polyvinyl ethers, hydroxypropylcellulose, polyvinylmorpholinone, and polymers and copolymers of vinyl sulfonic acid, polyacrylates, polyacrylamides, polyvinyl amines, polyallylamines, and polyvinylpyrridine.

10. The method of claim 1, wherein the superabsorbent material is selected from the group consisting of agar, algin, carrageenan, starch, pectin, guar gum, chitosan, and the like, modified natural materials such as carboxyalkyl cellulose, methyl cellulose, hydroxyalkyl cellulose, chitosan salt, and dextran.

11. The method of claim 1, wherein the surfactant first reactive functional group is selected from the group consisting of quaternary ammonium groups, amino groups, carboxyl groups, sulfonate groups, phosphate groups, and their corresponding acid groups.

12. The method of claim 1, wherein the surfactant non-reactive, hydrophilic functional group is selected from the group consisting of hydroxyl groups, ether groups, carboxylic acid groups, amino groups, and imino groups.

13. The method of claim 2, wherein the solvent is selected from the group consisting of isopropanol, methanol, ethanol, butyl alcohol, butanediol, butanetriol, butanone, acetone, ethylene glycol, propylene glycol, glycerol, and mixtures thereof.

14. The method of claim 2, wherein the water is present from about 1 to 10% by total weight of the solvent.

15. A disposable absorbent product comprising a liquid-permeable topsheet, a backsheet attached to the topsheet, and an absorbent structure with the fiber of claim 8 positioned between the topsheet and the backsheet.

16. The method of claim 1, wherein the surfactant is applied to the superabsorbent material when the superabsorbent material is in a solvated state.

## Patentansprüche

1. Verfahren zum Herstellen eines permanent benetzbaren, hoch absorbierenden Materials, das aufweist:
Behandeln des hoch absorbierenden Materials mit einer Lösung eines oberflächenaktiven Mittels;
wobei das oberflächenaktive Mittel mindestens eine erste, funktionale Gruppe, reaktiv mit einer zweiten, funktionalen Gruppe, des hoch absorbierenden Materials und mindestens eine nicht reaktive und hydrophile, funktionale Gruppe besitzt; und
wobei das oberflächenaktive Mittel auf das hoch absorbierende Material aufgebracht wird, wenn die zweiten, funktionalen Gruppen auf der Oberfläche des hoch absorbierenden Materials aktiviert sind.

2. Verfahren nach Anspruch 1, wobei die Lösung aus oberflächenaktivem Mittel ein Lösungsmittel umfasst, das ein Lösungsmittel für das oberflächenaktive Mittel, allerdings kein Lösungsmittel für das hoch absorbierende Material, ist; und
wobei die Lösung aus oberflächenaktivem Mittel eine Menge an Wasser umfasst, ausreichend, um die Oberfläche des hoch absorbierenden Materials zu solvatieren, allerdings geringer als ausreichend, um ein wesentliches Quellen des hoch absorbierenden Materials zu bewirken.

3. Verfahren nach Anspruch 2, das weiterhin ein Trocknen des behandelten, hoch absorbierenden Materials aufweist, um das Lösungsmittel und das Wasser zu entfernen.

4. Verfahren nach Anspruch 1, das weiterhin ein Waschen des behandelten, hoch absorbierenden Materials mit einem Lösungsmittel aufweist, um flüchtiges, oberflächenaktives Mittel zu entfernen.

5. Verfahren nach Anspruch 2, wobei die Behandlung durch Eintauchen oder Sprühen erfolgt.

6. Verfahren nach Anspruch 1, wobei das hoch absorbierende Material eine hoch absorbierende Faser ist.

7. Verfahren nach Anspruch 1, wobei das hoch absorbierende Material in einer Form vorliegt, die aus der Gruppe ausgewählt ist, die besteht aus Teilchen, einem Film einem Vlies, einem Wulst bzw. einer Perle, einem Schaum, und einer Coform.

8. Permanent benetzbare, hoch absorbierende Faser, die durch das Verfahren nach Anspruch 6 erhaltbar ist.

9. Verfahren nach Anspruch 1, wobei das hoch absorbierende Material aus der Gruppe ausgewählt ist, die besteht aus Alkalimetallsalzen von Polyacrylsäuren, Polyacrylamiden, Polyvinylalkohol, Ethylenmaleinanhydridcopolymeren, Polyvinylethern, Hydroxypropylzellulose, Polyvinylmorpholinon und Polymeren und Copolymeren aus Vinylsulfonsäure, Polyacrylaten, Polyacrylamiden, Polyvinylaminen, Polyallylaminen und Polyvinylpyrridin.

10. Verfahren nach Anspruch 1, wobei das hoch absorbierende Material aus der Gruppe ausgewählt ist, die aus Agar-Agar, Algin, Karrageen, Stärke, Pektin, Guar-Gummi, Chitosan, und dergleichen, modifizierten, natürlichen Materialien, wie beispielsweise Carboxyalkylzellulose, Methylzellulose, Hydroxyalkylzellulose, Chitosansalz und Dextran, besteht.

11. Verfahren nach Anspruch 1, wobei die erste, reaktive, funktionale Gruppe des oberflächenaktiven Mittels aus der Gruppe ausgewählt ist, bestehend aus quatemären Ammonium-Gruppen, Amino-Gruppen, Carboxyl-Gruppen, Sulfonat-Gruppen, PhosphatGruppen, und deren entsprechenden Säure-Gruppen.

12. Verfahren nach Anspruch 1, wobei die nicht-reaktive, hydrophile, funktionale Gruppe des oberflächenaktiven Mittels aus der Gruppe ausgewählt ist, die aus HydroxylGruppen, Ether-Gruppen, Carboxylsäure-Gruppen, Amino-Gruppen und Imino-Gruppen besteht.

13. Verfahren nach Anspruch 2, wobei das Lösungsmittel aus der Gruppe ausgewählt ist, die aus Isopropanol, Methanol, Ethanol, Butylalkohol, Butanediol, Butanetriol, Butanon, Aceton, Ethylenglycol, Propylenglycol, Glycerol, und Mischungen davon, besteht.

14. Verfahren nach Anspruch 2, wobei das Wasser von ungefähr 1 bis 10% bezogen auf das Gesamtgewicht des Lösungsmittels vorhanden ist.

15. Absorbierendes Einwegprodukt, das eine flüssigkeitsdurchlässige Oberseitenschicht, eine Unterlagenschicht, befestigt an der Oberseitenschicht, und eine absorbierende Struktur mit der Faser nach Anspruch 8 positioniert zwischen der Oberseitenschicht und der Unterlagenschicht aufweist.

16. Verfahren nach Anspruch 1, wobei das oberflächenaktive Mittel auf das hoch absorbierende Material dann aufgebracht wird, wenn sich das hoch absorbierende Material in einem solvatierten Zustand befindet.

## Revendications

1. Procédé de fabrication d'un matériau superabsorbant à mouillabilité permanente, comprenant :
le traitement du matériau superabsorbant avec une solution de tensioactif ;
le tensioactif ayant au moins un premier groupe fonctionnel pouvant réagir avec un second groupe fonctionnel du matériau superabsorbant et au moins un groupe fonctionnel non-réactif et hydrophile ; et
le tensioactif étant appliqué sur le matériau superabsorbant quand sont activés les seconds groupes fonctionnels à la surface du matériau superabsorbant.

2. Procédé selon la revendication 1, dans lequel la solution de tensioactif comprend un solvant qui est un solvant pour le tensioactif mais non un solvant pour le matériau superabsorbant ; et
dans lequel la solution de tensioactif comprend une quantité d'eau suffisante pour solvater la surface du matériau superabsorbant, mais inférieure à la quantité suffisante pour causer un gonflement significatif du matériau superabsorbant.

3. Procédé selon la revendication 2 comprenant, en outre, le séchage du matériau superabsorbant traité, pour éliminer le solvant et l'eau.

4. Procédé selon la revendication 1 comprenant, en outre, le lavage du matériau superabsorbant traité, avec un solvant pour éliminer le tensioactif fugitif.

5. Procédé selon la revendication 2, dans lequel le traitement se fait par immersion ou par pulvérisation.

6. Procédé selon la revendication 1, dans lequel le matériau superabsorbant est une fibre superabsorbante.

7. Procédé selon la revendication 1, dans lequel le matériau superabsorbant est sous une forme choisie dans le groupe consistant en une particule, un film, un non-tissé, une perle, une mousse et un coformé.

8. Fibre superabsorbante à mouillabilité permanente pouvant être obtenue par le procédé selon la revendication 6.

9. Procédé selon la revendication 1, dans lequel le matériau superabsorbant est choisi dans le groupe consistant en les sels de métaux alcalins des poly(acide acrylique), les polyacrylamides, le poly(alcool vinylique), les copolymères éthylène-anhydride maléique, les poly(vinyléthers), l'hydroxypropylcellulose, la polyvinylmorpholinone, les polymères et les copolymères de l'acide vinylsulfonique, les polyacrylates, les polyacrylamides, les polyvinylamines, les polyallylamines et la palyvinylpyridine.

10. Procédé selon la revendication 1, dans lequel le matériau superabsorbant est choisi dans le groupe consistant en l'agar, l'alginate de sodium, le carraghénane, l'amidon, la pectine, la gomme de guar, le chitosan, et analogues, les matériaux naturels modifiés tels que la carboxyalkylcellulose, la méthylcellulose, l'hydroxyalkylcellulose, le sel de chitosan et le dextran.

11. Procédé selon la revendication 1, dans lequel le premier groupe fonctionnel réactif du tensioactif est choisi dans le groupe consistant en les groupes ammonium quaternaire, les groupes amino, les groupes carboxyle, les groupes sulfonate, les groupes phosphate, et les groupes acides qui leur correspondent.

12. Procédé selon la revendication 1, dans lequel le groupe fonctionnel non réactif, hydrophile, du tensioactif, est choisi dans le groupe consistant en les groupes hydroxyle, les groupes éther, les groupes acide carboxylique, les groupes amino et les groupes imino.

13. Procédé selon la revendication 2, dans lequel le solvant est choisi dans le groupe consistant en l'isopropanol, le méthanol, l'éthanol, le butanol, le butanediol, le butanetriol, la butanone, l'acétone, l'éthylène glycol, le propylène glycol, le glycérol et leurs mélanges.

14. Procédé selon la revendication 2, dans lequel l'eau est présente à raison d'environ 1 à 10% du poids total du solvant.

15. Produit absorbant jetable comprenant une feuille supérieure perméable aux liquides, une feuille dossier fixée à la feuille supérieure, et une structure absorbante avec la fibre selon la revendication 8 positionnée entre la feuille supérieure et la feuille dossier.

16. Procédé selon la revendication 1, dans lequel le tensioactif est appliqué au matériau superabsorbant quand le matériau superabsorbant est à l'état solvaté.
